# EUROPEAN PATENT APPLICATION

(11) **EP 4 252 627 A1**
(43) Date of publication of application: **04.10.2023**
(21) Application number: 23165391.6
(22) Date of filing: 30.03.2023
(51) Int. Cl.: A61B 3/103, A61B 3/036, A61B 3/15, A61B 3/08

(54) **OPTOMETRY APPARATUS AND OPTOMETRY PROGRAM**

(30) Priority: 31.03.2022 JP 2022059856
(71) Applicant: Nidek Co., Ltd., Gamagori-Aichi, 443-0038 (JP)
(72) Inventor: KAMIKAWA, Tomohiro, Gamagori, Aichi (JP)
(74) Representative: Hoefer & Partner Patentanwälte mbB

(57) **Abstract**

An optometry apparatus measures optical characteristics of a subject eye. The optometry apparatus includes an objective measurement optical system that objectively measures the optical characteristics of the subject eye, a subjective measurement optical system that subjectively measures the optical characteristics of the subject eye, a first control means that performs first alignment processing based on a first alignment allowable range for determining a deviation between the subject eye and the objective measurement optical system, and a second control means that performs second alignment processing based on a second alignment allowable range for determining a deviation between the subject eye and the subjective measurement optical system. The second alignment allowable range has a wider allowable range than the first alignment allowable range.

## Description

### TECHNICAL FIELD

The present disclosure relates to an optometry apparatus which measures optical characteristics of a subject eye, and an optometry program executed by a processor of the optometry apparatus.

### BACKGROUND ART

An optometry apparatus is known that includes both an objective measurement optical system for objectively measuring optical characteristics of a subject eye and a subjective measurement optical system for subjectively measuring the optical characteristics of the subject eye (for example, refer to JP2018-047050A). In such an optometry apparatus, subjective measurement using the subjective measurement optical system is performed after objective measurement using the objective measurement optical system.

When measuring the subject eye, alignment is performed to adjust a positional relationship between the subject eye and the optometry apparatus. For example, an allowable range is provided for determining a deviation between the subject eye and the optometry apparatus, and alignment is performed as appropriate, in a case where the deviations are separated from each other beyond the allowable range. However, even though the measurement on the subject eye is performed based on this allowable range, the measurement result of the optical characteristics of the subject eye may not be obtained satisfactorily.

### SUMMARY OF INVENTION

A technical object of the present disclosure is to provide an optometry apparatus and an optometry program that can satisfactorily measure optical characteristics of a subject eye.
(1) An optometry apparatus that measures optical characteristics of a subject eye, having:
   an objective measurement optical system including a first light projecting optical system that projects a measurement light flux onto a fundus of the subject eye, and a light receiving optical system in which a detector detects a reflection light flux, reflected by the fundus, of the measurement light flux, and for objectively measuring the optical characteristics of the subject eye;
   a subjective measurement optical system including a second light projecting optical system that projects a target light flux toward the subject eye, and a calibration optical system that is disposed in an optical path of the second light projecting optical system and changes optical characteristics of the target light flux, and for subjectively measuring the optical characteristics of the subject eye;
   a first control means configured to perform first alignment processing based on a first alignment allowable range for determining a deviation between the subject eye and the objective measurement optical system; and
   a second control means configured to perform second alignment processing based on a second alignment allowable range for determining a deviation between the subject eye and the subjective measurement optical system,
   in which the second alignment allowable range has a wider allowable range than the first alignment allowable range.
(2) The optometry apparatus according to the above-described (1),
   in which the first control means is configured to adjust a relative positional relationship between the subject eye and the objective measurement optical system as the first alignment processing, and
   the second control means is configured to adjust a relative positional relationship between the subject eye and the subjective measurement optical system as the second alignment processing.
(3) The optometry apparatus according to the above-described (1) or (2), further having:
   a change control means configured to change between the first alignment allowable range corresponding to objective measurement and the second alignment allowable range corresponding to subjective measurement, when measurement on the subject eye is switched from one of the objective measurement by the objective measurement optical system and the subjective measurement by the subjective measurement optical system to the other.
(4) The optometry apparatus according to the above-described (3),
   in which the change control means sets the second alignment allowable range, in a case where the optical characteristics of the subject eye is objectively measured by the objective measurement optical system while the optical characteristics of the subject eye is subjectively measured by the subjective measurement optical system.
(5) The optometry apparatus according to any one of the above-described (1) to (4), further having:
   a measurement unit including the objective measurement optical system and the subjective measurement optical system, and having a pair of a left eye measurement unit and a right eye measurement unit,
   in which the left eye measurement unit is aligned with a left eye and the right eye measurement unit is aligned with a right eye, respectively.
(6) The optometry apparatus according to the above-described (5),
   in which the left eye measurement unit projects the target light flux onto the left eye and the right eye measurement unit projects the target light flux onto the right eye, the left eye and the right eye being subjected to a binocular fusion.
(7) The optometry apparatus according to any one of the above-described (1) to (6), further having:
   a shared optical member shared by an optical path of the objective measurement optical system and an optical path of the subjective measurement optical system, and for optically presenting an image of the target light flux to the subject eye at a predetermined test distance by guiding the target light flux corrected by the calibration optical system to the subject eye.
(8) An optometry program executed by a processor of an optometry apparatus that includes: an objective measurement optical system including a first light projecting optical system that projects a measurement light flux onto a fundus of a subject eye, and a light receiving optical system, in which a detector detects a reflection light flux, reflected by the fundus, of the measurement light flux, and for objectively measuring optical characteristics of the subject eye; and a subjective measurement optical system including a second light projecting optical system that projects a target light flux toward the subject eye, and a calibration optical system that is disposed in an optical path of the second light projecting optical system and changes optical characteristics of the target light flux, and for subjectively measuring the optical characteristics of the subject eye, and measures the optical characteristics of the subject eye, the optometry program including instructions which, when executed by the processor of the optometry apparatus, cause the optometry apparatus to perform:
   a first control step of performing first alignment processing based on a first alignment allowable range for determining a deviation between the subject eye and the objective measurement optical system; and
   a second control step of performing second alignment processing based on a second alignment allowable range for determining a deviation between the subject eye and the subjective measurement optical system,
   in which the second alignment allowable range has a wider allowable range than the first alignment allowable range.

### BRIEF DESCRIPTION OF DRAWINGS

FIG. 1 is an external view of an optometry apparatus.
FIG. 2 is a diagram illustrating a left eye measuring unit.
FIG. 3 is a schematic diagram of an inside of the optometry apparatus viewed from the front direction.
FIG. 4 is a schematic diagram of the inside of the optometry apparatus viewed from the side direction.
FIG. 5 is a schematic diagram of the inside of the optometry apparatus viewed from the top surface direction.
FIG. 6 is a diagram illustrating a control system.
FIG. 7 is an example of an anterior chamber image of a left eye.
FIG. 8 is a diagram for describing an alignment allowable range (alignment tolerance) for objective measurement.
FIG. 9 is a diagram for describing an alignment allowable range (alignment tolerance) for subjective measurement.

### DESCRIPTION OF EMBODIMENTS

### <Overview>

An outline of an optometry apparatus according to an embodiment of the present disclosure will be described. In the present embodiment, the horizontal direction of the optometry apparatus is the X direction, the vertical direction is the Y direction, and the front-rear direction (working distance direction) is the Z direction. Each of L and R attached to the sign indicates a left eye and a right eye. The items classified by < > below can be used independently or in association with each other.

The optometry apparatus of the present embodiment is an apparatus capable of measuring optical characteristics of a subject eye. For example, the optometry apparatus may be provided with an objective measurement optical system for objectively measuring the optical characteristics of the subject eye. For example, the optometry apparatus may be provided with a subjective measurement optical system for subjectively measuring the optical characteristics of the subject eye.

### <Objective Measurement Optical System>

The optometry apparatus of the present embodiment may be provided with the objective measurement optical system (for example, objective measurement optical system 10). For example, the objective measurement optical system may include a first light projecting optical system (for example, projection optical system 10a) that projects a measurement light flux onto a fundus of the subject eye, and a light receiving optical system (for example, light receiving optical system 10b), in which a detector receives a reflection light flux, reflected by the fundus, of the measurement light flux. As objective optical characteristics, at least one of eye refractive power (for example, spherical power, cylindrical power, astigmatism axis angle, and the like), eye axial length, corneal shape, and the like may be measured.

The first light projecting optical system may have a first light projecting optical system for the left eye and a first light projecting optical system for the right eye, which are provided as a pair on left and right. For example, each of the first light projecting optical system for the left eye and the first light projecting optical system for the right eye may be configured to include the same member, or at least a part of the members may be configured to include different members. For example, the first light projecting optical system for the left eye and the first light projecting optical system for the right eye may be configured such that at least a part of the members constituting each of the first light projecting optical systems are shared.

The first light receiving optical system may have a first light receiving optical system for the left eye and a first light receiving optical system for the right eye, which are provided as a pair on left and right. For example, each of the first light receiving optical system for the left eye and the first light receiving optical system for the right eye may be configured to include the same member, or at least a part of the members may be configured to include different members. For example, the first light receiving optical system for the left eye and the first light receiving optical system for the right eye may be configured such that at least a part of the members constituting each of the first light receiving optical systems are shared.

### <Subjective Measurement Optical System>

The optometry apparatus of the present embodiment may be provided with subjective measurement means (for example, subjective measurement optical system 25). For example, the subjective measurement optical system may include a second light projecting optical system (for example, light projecting optical system 30) that projects a target light flux toward the subject eye, and a calibration optical system (for example, calibration optical system 60) disposed in an optical path of the second light projecting optical system and changing the optical characteristics of the target light flux. As subjective optical characteristics, at least one of eye refractive power (spherical power, cylindrical power, astigmatism axis angle, and the like), contrast sensitivity, binocular vision function (amount of oblique vision, stereoscopic vision function, and the like), and the like may be measured.

### <Second Light Projecting Optical System>

The second light projecting optical system projects a target light flux toward the subject eye. The second light projecting optical system may include at least one optical member that guides the target light flux toward the subject eye.

The second light projecting optical system may be provided with target presentation means. The target presentation means presents a target to the subject eye. In this case, the second light projecting optical system projects the target light flux emitted from the target presentation means toward the subject eye. For example, a display (for example, display 31) can be used as the target presentation means. For example, a light source and a digital micromirror device (DMD) can be used as the target presentation means. In general, the DMD has a high reflectance and is bright, so it is possible to maintain the light amount of target light flux more than using an LCD. For example, a visible light source and a target plate for target presentation can be used as the target presentation means. The target plate is a rotatable disc plate and may have a plurality of targets. The target plate is rotated by a motor or the like on the optical path along which the target light flux is guided to the subject eye, so that the targets are switched and disposed.

The second light projecting optical system may have a second light projecting optical system for the left eye and a second light projecting optical system for the right eye, which are provided as a pair on left and right. For example, each of the second light projecting optical system for the left eye and the second light projecting optical system for the right eye may be configured to include the same member, or at least a part of the members may be configured to include different members. For example, the second light projecting optical system for the left eye and the second light projecting optical system for the right eye may be configured such that at least a part of the members constituting each of the second light projecting optical systems are shared.

### <Calibration Optical System>

The calibration optical system is disposed in the optical path of the second light projecting optical system, and changes the optical characteristics of the target light flux (at least one of a spherical power, a cylindrical power, an astigmatism axis angle, polarization characteristics, the amount of aberration, and the like).

For example, the calibration optical system may be capable of changing at least one of the spherical power, the cylindrical power, the astigmatism axis angle, and the like of the target light flux by controlling the optical element. The optical element may be at least one of a spherical lens, a cylindrical lens, a cross-cylinder lens, a rotary prism, a wave front modulating element, a variable focus lens, and the like. As a matter of course, optical elements different from these optical elements may be used.

For example, the calibration optical system may correct the spherical power of the subject eye by optically changing the presentation distance of the target with respect to the subject eye. In this case, the target presentation means may be moved in the optical axis direction in order to optically change the presentation distance of the target. In this case, in order to optically change the presentation distance of the target, an optical element (for example, a spherical lens or the like) disposed in the optical path may be moved in the optical axis direction.

The calibration optical system may be configured to be a combination of a configuration for controlling the optical element, a configuration for moving the target presentation means in the optical axis direction, and a configuration for moving the optical element disposed in the optical path in the optical axis direction.

The calibration optical system may be configured to change the optical characteristics of the target light flux by disposing an optical element between the target presentation means and the optical member for guiding the target light flux from the second light projecting optical system toward the subject eye, and controlling the optical element. That is, the calibration optical system may be configured as a phantom lens refractometer (phantom calibration optical system). In this case, the target light flux corrected by the calibration optical system is guided to the subject eye via the optical member.

The calibration optical system may have a calibration optical system for the left eye and a calibration optical system for the right eye, which are provided as a pair on left and right. For example, each of the calibration optical system for the left eye and the calibration optical system for the right eye may be configured to include the same member, or at least a part of the members may be configured to include different members. For example, the calibration optical system for the left eye and the calibration optical system for the right eye may be configured such that at least a part of the members constituting each of the calibration optical systems are shared.

### <Measurement Unit>

The optometry apparatus of the present embodiment may be provided with a measurement unit (for example, measuring unit 7). The measurement unit includes the objective measurement optical system and the subjective measurement optical system, and has a pair of a left eye measurement unit (for example, left eye measuring unit 7L) and a right eye measurement unit (for example, right eye measuring unit 7R). That is, the left eye measurement unit including an objective measurement optical system for the left eye and a subjective measurement optical system for the left eye, the right eye measurement unit including an objective measurement optical system for the right eye and a subjective measurement optical system for the right eye are provided. The subjective measurement optical system for the left eye has a second light projecting optical system for the left eye and a calibration optical system for the left eye. The subjective measurement optical system for the right eye has a second light projecting optical system for the right eye and a calibration optical system for the right eye. For example, with such a configuration, the left eye measurement unit can be aligned with the left eye, and the right eye measurement unit can be aligned with the right eye, respectively.

The optometry apparatus of the present embodiment has the pair of the left eye measurement unit and the right eye measurement unit. Therefore, the target light flux can be projected from the second light projecting optical system in the left eye measurement unit onto the left eye, and the target light flux can be projected from the second light projecting optical system in the right eye measurement unit onto the right eye. As a result, a binocular fusion of the left eye and the right eye can be performed.

### <Shared Optical Member>

The optometry apparatus of the present embodiment may be provided with a shared optical member. The shared optical member is an optical member shared by the optical path of the objective measurement optical system and the optical path of the subjective measurement optical system. For example, the shared optical member may guide the measurement light flux from the first light projecting optical system in the objective measurement optical system to the subject eye. The shared optical member may guide the target light flux projected from the second light projecting optical system in the subjective measurement optical system and corrected by the calibration optical system to the subject eye. As a result, an image of the target light flux may be optically presented to the subject eye at a predetermined test distance.

The shared optical member may be an optical member fixedly disposed in the optical path of the first light projecting optical system in the objective measurement optical system. The shared optical member may be an optical member that is fixedly disposed in the optical path of the second light projecting optical system in the subjective measurement optical system. The shared optical member may be shared by the optical path of the measurement light flux and the target light flux from the left eye measurement unit and the optical path of the measurement light flux and the target light flux from the right eye measurement unit. For example, at least one of a concave mirror (for example, concave mirror 85), a lens, and the like may be used as the shared optical member.

### <Alignment Allowable Range (Alignment Tolerance) >

The optometry apparatus of the present embodiment may have a first alignment allowable range (for example, alignment allowable range B1). The first alignment allowable range is a tolerance for determining a deviation between the subject eye and the objective measurement optical system. For example, the first alignment allowable range may be an allowable range for determining a deviation in the X direction between the subject eye and the objective measurement optical system. It may be an allowable range for determining a deviation in the Y direction between the subject eye and the objective measurement optical system. It may be an allowable range for determining a deviation in the Z direction between the subject eye and the objective measurement optical system. As a matter of course, the allowable range may be a combination of these a plurality of directions.

The first alignment allowable range may be an allowable range for determining a deviation between the subject eye and the first light projecting optical system in the objective measurement optical system. It may be an allowable range for determining a deviation between the subject eye and the light receiving optical system in the objective measurement optical system. As a matter of course, it may be an allowable range for determining a deviation between the subject eye and both the first light projecting optical system and the light receiving optical system.

The optometry apparatus of the present embodiment may have a second alignment allowable range (for example, alignment allowable range B2). The second alignment allowable range is a tolerance for determining a deviation between the subject eye and the subjective measurement optical system. For example, the second alignment allowable range may be an allowable range for determining a deviation in the X direction between the subject eye and the subjective measurement optical system. It may be an allowable range for determining a deviation in the Y direction between the subject eye and the subjective measurement optical system. It may be an allowable range for determining a deviation in the Z direction between the subject eye and the subjective measurement optical system. As a matter of course, the allowable range may be a combination of these a plurality of directions.

The second alignment allowable range may be an allowable range for determining a deviation between the subject eye and the subjective measurement optical system such that a target is presented to the subject eye. For example, it may be in the same range as the typical pupil diameter or in the range smaller than the typical pupil diameter. As a result, at least a part of the target light flux from the second light projecting optical system directed to the subject eye is vignetted, and the possibility that the target appears to be missing from the subject eye is reduced.

At least one of the first alignment allowable range and the second alignment allowable range may be an allowable range of fixed value set in advance. At least one of the first alignment allowable range and the second alignment allowable range may be an allowable range of any value set based on an operation signal for setting each allowable range. In this case, for example, the operation signal may be output when an examiner operates operation means (for example, switch unit 6b). For example, the operation signal may be output by using external storage means (as an example, SD card, USB memory, server, cloud, and the like) and reading data stored in the external storage means.

In the present embodiment, the second alignment allowable range is set to have a wider allowable range than the first alignment allowable range. For example, the second alignment allowable range may be set in a range that is wider than the first alignment allowable range and is substantially the same as the typical pupil diameter (or a range smaller than the typical pupil diameter). As a result, each measurement is performed in an alignment allowable range suitable for each of the objective measurement using the objective measurement optical system and the subjective measurement using the subjective measurement optical system for the subject eye. For example, in the objective measurement, the deviation between the subject eye and the objective measurement optical system is finely adjusted, so that measurement results can be obtained with high accuracy. In the subjective measurement, the deviation between the subject eye and the subjective measurement optical system is allowed to some extent, the frequency of the target appearing to move decreases. Therefore, the subject is unlikely to feel annoyance and fatigue, and good measurement results can be obtained. Even in a case where the subject eye moves significantly and is deviated from the second alignment allowable range, the deviation is adjusted as appropriate, so the target is appropriately presented to the subject eye.

### <Control Means>

The optometry apparatus of the present embodiment may be provided with first control means (for example, control section 70). The first control means performs first alignment processing based on the first alignment allowable range for determining the deviation between the subject eye and the objective measurement optical system. The first control means may perform , as the first alignment processing, processing capable of eliminating the deviation between the subject eye and the objective measurement optical system. For example, the relative positional relationship between the subject eye and the objective measurement optical system may be adjusted. As an example, the objective measurement optical system may be moved with respect to the subject eye. For example, in order to dispose the subject eye at an appropriate position, face support means (for example, at least one of the forehead rest 4, the chin rest 5, the cheek rest, and the like) that supports at least a part of the face of the subject may be operated. For example, guide information for guiding the subject eye to an appropriate position may be output. As an example, a message or the like telling the subject to move his or her face may be output by at least one of control of sound generation means, control of display means, and the like.

The optometry apparatus of the present embodiment may be provided with second control means (for example, control section 70). The second control means performs second alignment processing based on the second alignment allowable range for determining the deviation between the subject eye and the subjective measurement optical system. The second control means may perform, as the second alignment processing, processing capable of eliminating the deviation between the subject eye and the subjective measurement optical system. For example, the relative positional relationship between the subject eye and the subjective measurement optical system may be adjusted. As an example, the subjective measurement optical system may be moved with respect to the subject eye. For example, similarly to the first control means, the face support means may be operated to dispose the subject eye at an appropriate position, or guide information for guiding the subject eye to an appropriate position may be output.

In the present embodiment, the first control means adjusts the relative positional relationship between the subject eye and the objective measurement optical system as the first alignment processing. For example, the objective measurement optical system may be moved at least in the X and Y directions. As a matter of course, for example, the objective measurement optical system may be moved in the Z direction. In a case where the objective measurement optical system deviates from the subject eye by exceeding the first alignment allowable range, the first control means may perform tracking control so that the objective measurement optical system is aligned with the subject eye.

In the present embodiment, the second control means adjusts the relative positional relationship between the subject eye and the subjective measurement optical system as the second alignment processing. For example, the subjective measurement optical system may be moved at least in the X and Y directions. As a matter of course, for example, the subjective measurement optical system may be moved in the Z direction. For example, in a case where the subjective measurement optical system deviates from the subject eye by exceeding the second alignment allowable range, the second control means may perform tracking control so that the subjective measurement optical system is aligned with the subject eye.

For example, since the subject eye is likely to deviate in the XY directions, by moving the objective measurement optical system and the subjective measurement optical system in at least the XY directions, the possibility that the target cannot be presented appropriately to the subject eye is reduced. By moving the objective measurement optical system and the subjective measurement optical system in the three-dimensional direction with respect to the subject eye, the objective measurement optical system and the subjective measurement optical system can be quickly disposed at appropriate positions according to the deviation of the subject eye.

The first control means and the second control means may be used in common. That is, by using one moving means, alignment processing based on the first alignment allowable range between the subject eye and the objective measurement optical system and alignment processing based on the second alignment allowable range between the subject eye and the subjective measurement optical system may be configured to be performed.

### <Change Control Means>

The optometry apparatus of the present embodiment may be provided with change control means (for example, control section 70). When the measurement on the subject eye is switched from one of the objective measurement by the objective measurement optical system and the subjective measurement by the subjective measurement optical system to the other, the change control means changes between the first alignment allowable range corresponding to the objective measurement and the second alignment allowable range corresponding to the subjective measurement. For example, when switching from the objective measurement to the subjective measurement on the subject eye, the setting may be changed from the first alignment allowable range to the second alignment allowable range. For example, when switching from the subjective measurement to the objective measurement on the subject eye, the setting may be changed from the second alignment allowable range to the first alignment allowable range. As a result, each measurement on the subject eye can be proceeded smoothly, and measurement results can be obtained with high accuracy.

When the measurement on the subject eye is switched from the objective measurement to the subjective measurement, the change control means may change the setting from the first alignment allowable range to the second alignment allowable range at any timing from the start of the subjective measurement to the end thereof. When the measurement on the subject eye is switched from the subjective measurement to the objective measurement, the change control means may change the setting from the second alignment allowable range to the first alignment allowable range at any timing from the start of the objective measurement to the end thereof.

The change control means may set each allowable range based on a change signal for changing between the first alignment allowable range and the second alignment allowable range. For example, the change signal may be output when the examiner operates the operation means. As an example, the change signal may be output using an operation for starting at least one of the objective measurement and the subjective measurement, an operation for specifying at least one of the type of target to be presented to the subject eye and a visual acuity value, an operation for specifying a correction power for correcting the subject eye, or the like as a trigger. For example, the change signal may be output based on a program for automatically proceeding with at least one of the objective measurement and the subjective measurement.

The change control means may set the second alignment allowable range, in a case where the optical characteristics of the subject eye is objectively measured by the objective measurement optical system while the optical characteristics of the subject eye is subjectively measured by the subjective measurement optical system. For example, the optical characteristics of the subject eye may be objectively measured once while the optical characteristics of the subject eye is subjectively measured. For example, the optical characteristics of the subject eye may be objectively measured a plurality of times while the optical characteristics of the subject eye is subjectively measured. As an example, a plurality of measurements may be performed at least one timing such as each time a predetermined time elapses after the start of the subjective measurement, each time the type of target presented to the subject eye is switched, each time the visual acuity value of the target presented on the subject eye is switched, each time the correction power for correcting the subject eye is switched, and the like. For example, the optical characteristics of the subject eye may be objectively measured all the time (in real time) while the optical characteristics of the subject eye is subjectively measured. As a result, both objective measurement results and subjective measurement results for the subject eye can be acquired satisfactorily.

The present disclosure is not limited to the apparatus described in the present embodiment. For example, the terminal control software (program) that executes the instructions of the above embodiments can be supplied to a system or an apparatus via a network or various storage media, and the like, so that the control device (for example, CPU and the like) of the system or the apparatus can read and execute the program.

### <Example>

An example of an optometry apparatus (hereinafter referred to as an optometry apparatus) according to the present embodiment will be described.

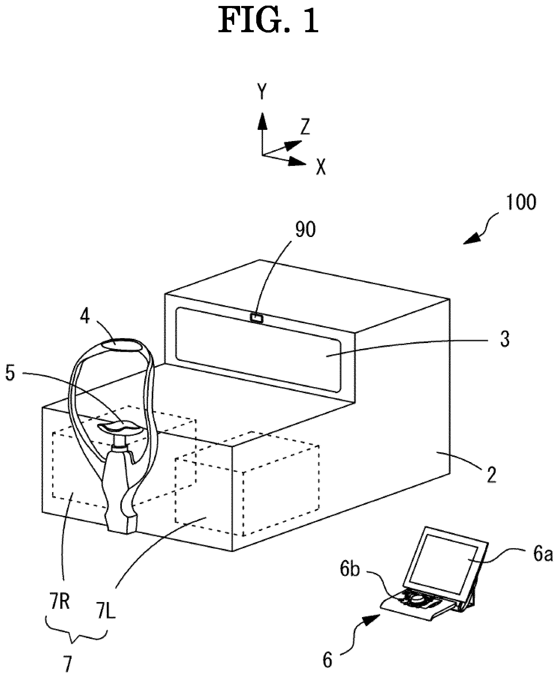

FIG. 1 is an external view of an optometry apparatus 100. For example, the optometry apparatus 100 is provided with a housing 2, a presentation window 3, a forehead rest 4, a chin rest 5, a controller 6, an imaging unit 90, and the like. The housing 2 has a measuring unit 7, a deflection mirror 81, a reflection mirror 84, a concave mirror 85, and the like inside thereof. The presentation window 3 is used to present the target to a subject eye E. The forehead rest 4 and chin rest 5 are used to keep the distance between the subject eye E and the optometry apparatus 100 constant. The controller 6 includes a monitor 6a, a switch unit 6b, and the like. The monitor 6a displays various types of information (for example, measurement results of the subject eye, and the like). The monitor 6a may be a touch panel that also functions as the switch unit 6b. The switch unit 6b is used to perform various settings (for example, input of a start signal, and the like). A signal corresponding to an operation instruction from the controller 6 is output to the control section 70 through wired communication or wireless communication.

The imaging unit 90 is used to capture an image of the face of the subject and adjust the position of the subject eye in the Y direction. The imaging unit 90 is provided with an imaging optical system (not illustrated). For example, the imaging optical system may be configured to include an imaging element and a lens.

### <Measuring Unit>

The measuring unit 7 is provided with a left eye measuring unit 7L and a right eye measuring unit 7R. The left eye measuring unit 7L and the right eye measuring unit 7R are made of the same member. As a matter of course, at least a part of the left eye measuring unit 7L and the right eye measuring unit 7R may be configured to include different members. The measuring unit 7 includes a pair of left and right subjective measuring units and a pair of left and right objective measuring units (details will be described later). A target light flux and a measurement light flux from the measuring unit 7 are guided to the subject eye E through the presentation window 3.

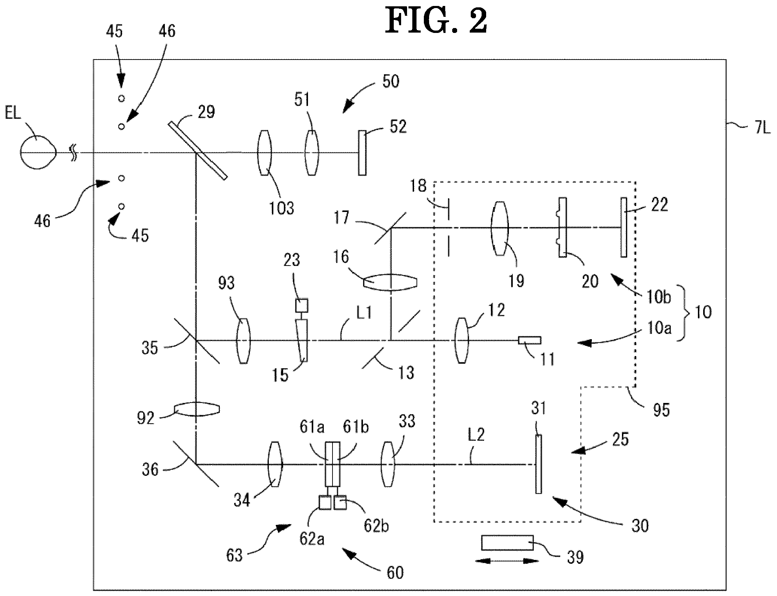

FIG. 2 is a diagram illustrating the left eye measuring unit 7L. The right eye measuring unit 7R will be omitted because the right eye measuring unit 7R has the same configuration as that of the left eye measuring unit 7L. For example, the left eye measuring unit 7L is provided with an objective measurement optical system 10, a subjective measurement optical system 25, a first index projection optical system 45, a second index projection optical system 46, an observation optical system 50, and the like.

### <Objective Measurement Optical System>

The objective measurement optical system 10 is used as a part of the configuration of the objective measuring unit that objectively measures the optical characteristics of the subject eye. In the present example, the eye refractive power of the subject eye E is measured as the optical characteristics of the subject eye E. For example, the objective measurement optical system 10 is configured to include a projection optical system 10a and a light receiving optical system 10b.

The projection optical system 10a projects a spot-like measurement target onto the fundus of the subject eye E through the center of the pupil of the subject eye E. For example, the projection optical system 10a is provided with a light source 11, a relay lens 12, a hole mirror 13, a prism 15, an objective lens 93, a dichroic mirror 35, a dichroic mirror 29, and the like. The light source 11 emits a measurement light flux. The light source 11 has a conjugate relationship with the fundus of the subject eye E. A hole unit of the hole mirror 13 is in a conjugate relationship with the pupil of the subject eye E. The prism 15 is a flux deflection member. The prism 15 is disposed at a position deviated from a position conjugated with the pupil of the subject eye E, and causes the measurement light flux passing through the prism 15 to be eccentric with respect to an optical axis L1. The prism 15 is rotationally driven by a drive unit (motor) 23 about the optical axis L1. The dichroic mirror 35 makes the optical path of the objective measurement optical system 10 and the optical path of the subjective measurement optical system 25 a common optical path. That is, the optical axis L1 of the objective measurement optical system 10 and an optical axis L2 of the subjective measurement optical system 25 are coaxial. The dichroic mirror 29 is an optical path branching member. The dichroic mirror 29 reflects the measurement light flux from the projection optical system 10a and the target light flux from the light projecting optical system 30 (described later) and guides these light flux to the subject eye E.

The light receiving optical system 10b takes out the fundus reflection light flux reflected by the fundus of the subject eye E in a ring shape through the periphery of the pupil of the subject eye E. For example, the light receiving optical system 10b is provided with a dichroic mirror 29, a dichroic mirror 35, an objective lens 93, a prism 15, a hole mirror 13, a relay lens 16, a mirror 17, a light receiving diaphragm 18, a collimator lens 19, a ring lens 20, an imaging element 22, and the like. The ring lens 20 is configured to include a ring-shaped lens unit and a light shielding unit having a light shielding coating applied to a region other than the lens unit. The ring lens 20 is in an optically conjugate positional relationship with the pupil of the subject eye E. The light receiving diaphragm 18 and the imaging element 22 are in a conjugate relationship with the fundus of the subject eye E. An output from the imaging element 22 is input to the control section 70.

In the present example, the prism 15 is disposed on the common optical axis of the projection optical system 10a and the light receiving optical system 10b. For example, the measurement light flux from the projection optical system 10a passes through the prism 15 and enters the subject eye E, and the fundus reflection light flux reflected by the fundus of the subject eye E passes through the same prism 15. Therefore, in the subsequent optical system, reverse scanning is performed as if there was no eccentricity of the projected light flux and the fundus reflection light flux (received light flux) on the pupil.

### <Subjective Measurement Optical System>

The subjective measurement optical system 25 is used as a part of the configuration of the subjective measuring unit that subjectively measures the optical characteristics of the subject eye E. In the present example, the eye refractive power of the subject eye E is measured as the optical characteristics of the subject eye E. For example, the subjective measurement optical system 25 is configured to include a light projecting optical system 30 and a calibration optical system 60.

### <Light Projecting Optical System>

The light projecting optical system 30 projects a target light flux toward the subject eye E. For example, the light projecting optical system 30 is provided with a display 31, a projection lens 33, a projection lens 34, a reflection mirror 36, an objective lens 92, a dichroic mirror 35, a dichroic mirror 29, and the like. The display 31 displays targets (fixation target, test target, and the like).

### <Calibration Optical System>

The calibration optical system 60 is disposed in the optical path of the light projecting optical system 30. The calibration optical system 60 changes the optical characteristics of the target light flux emitted from the display 31. For example, the calibration optical system 60 is provided with an astigmatism calibration optical system 63, a drive mechanism 39, and the like. The astigmatism calibration optical system 63 is used to correct the cylindrical power and astigmatism axis angle of the subject eye E. The astigmatism calibration optical system 63 is disposed between the projection lens 33 and the projection lens 34. The astigmatism calibration optical system 63 is configured to include two positive cylindrical lenses 61a and 61b having the same focal length. The cylindrical lens 61a and the cylindrical lens 61b are independently rotated about the optical axis L2 by driving a rotating mechanism 62a and a rotating mechanism 62b.

In the present example, the configuration using the cylindrical lenses 61a and 61b as the astigmatism calibration optical system 63 is described as an example, but the configuration is not limited thereto. The astigmatism calibration optical system 63 may have any configuration as long as the astigmatism calibration optical system 63 can correct the cylindrical power, astigmatism axis angle, and the like. As an example, a corrective lens may be inserted into or removed from the optical path of the light projecting optical system 30.

The light source 11 and the relay lens 12 included in the projection optical system 10a, the light receiving diaphragm 18, the collimator lens 19, the ring lens 20, and the imaging element 22 included in the light receiving optical system 10b, and the display 31 included in the light projecting optical system 30 are integrally movable in the optical axis direction by the drive mechanism 39. That is, the display 31, the light source 11, the relay lens 12, the light receiving diaphragm 18, the collimator lens 19, the ring lens 20, and the imaging element 22 are synchronized as the drive unit 95, and are moved integrally by the drive mechanism 39. The drive mechanism 39 includes a motor and a slide mechanism.

The drive mechanism 39 moves the display 31 in the direction of the optical axis L2 by moving the drive unit 95 in the optical axis direction. As a result, the subject eye E can be fogged in the objective measurement. In the subjective measurement, it is possible to optically change the presentation distance of the target with respect to the subject eye E to correct the spherical power of the subject eye E. That is, the configuration for moving the display 31 in the direction of the optical axis L2 is used as a spherical calibration optical system for correcting the spherical power of the subject eye E, and by changing the position of the display 31, the spherical power of the subject eye E is corrected. The configuration of the spherical calibration optical system may be different from that of the present example. For example, multiple optical elements may be disposed in the optical path to correct the spherical power. For example, the spherical power may be corrected by disposing a lens in the optical path and moving the lens in the optical axis direction.

The drive mechanism 39 moves the light source 11, the relay lens 12, and the imaging element 22 from the light receiving diaphragm 18 to in the direction of the optical axis L1 by moving the drive unit 95 in the optical axis direction. As a result, the light source 11, the light receiving diaphragm 18, and the imaging element 22 are disposed so as to be optically conjugate with respect to the fundus of the subject eye E. Regardless of the movement of the drive unit 95, the hole mirror 13 and the ring lens 20 are disposed so as to be conjugate with the pupil of the subject eye E at a constant magnification. Therefore, the fundus reflection light flux, reflected by the fundus, of the measurement light flux of the projection optical system 10a is normally incident on the ring lens 20 of the light receiving optical system 10b as a parallel light flux, and regardless of the eye refractive power of the subject eye E, a ring-shaped light flux having the same size as the ring lens 20 is captured by the imaging element 22 in a focused state.

### <First Index Projection Optical System and Second Index Projection Optical System>

The first index projection optical system 45 and the second index projection optical system 46 are disposed between the dichroic mirror 29 and a deflection mirror 81 (described later). The first index projection optical system 45 emits near-infrared light for projecting an infinite alignment index onto the cornea of the subject eye E. The second index projection optical system 46 is disposed at a position different from that of the first index projection optical system 45, and emits near-infrared light for projecting a finite alignment index onto the cornea of the subject eye. The near-infrared light (alignment light) emitted from the second index projection optical system 46 is also used as an anterior chamber imaging light for imaging the anterior chamber of the subject eye by the observation optical system 50.

### <Observation Optical System>

The observation optical system (imaging optical system) 50 is provided with a dichroic mirror 29, an objective lens 103, an imaging lens 51, an imaging element 52, and the like. The dichroic mirror 29 transmits the anterior chamber observation light and the alignment light. The imaging element 52 has an imaging plane disposed at a position conjugate with the anterior chamber of the subject eye E. An output from the imaging element 52 is input to the control section 70. As a result, the image of the anterior chamber of the subject eye E is captured by the imaging element 52 and displayed on the monitor 6a. The observation optical system 50 also serves as an optical system for detecting an alignment index image formed on the cornea of the subject eye E by the first index projection optical system 45 and the second index projection optical system 46, and the position of the alignment index image is detected by the control section 70.

### <Internal Configuration of Optometry Apparatus>

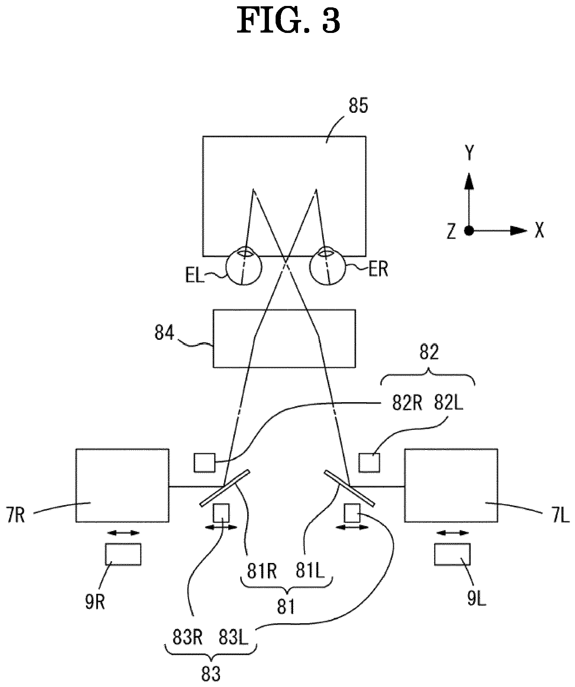

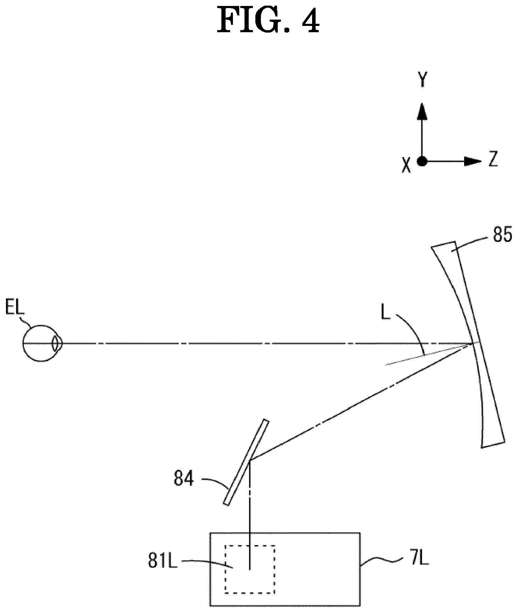

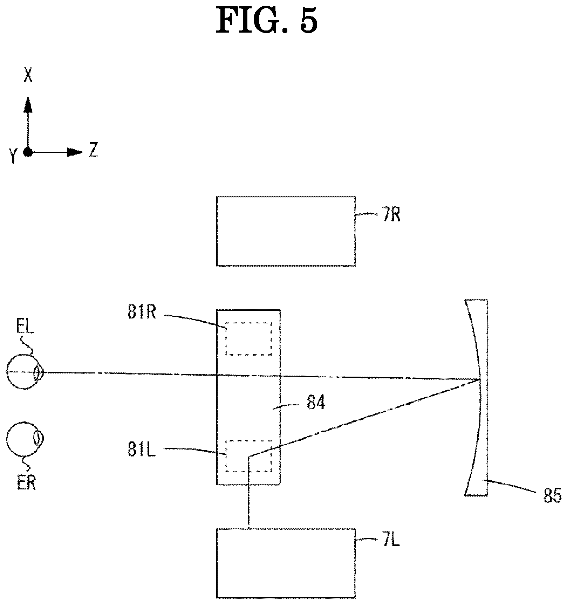

The internal configuration of the optometry apparatus 100 will be described. FIG. 3 is a schematic diagram of an inside of the optometry apparatus 100 viewed from the front direction. FIG. 4 is a schematic diagram of the inside of the optometry apparatus 100 viewed from the side direction. FIG. 5 is a schematic diagram of the inside of the optometry apparatus 100 viewed from the top surface direction. FIGS. 4 and 5 illustrate only the optical axis of the left eye measuring unit 7L for convenience of description.

The optometry apparatus 100 is provided with an objective measuring unit. For example, the objective measuring unit is configured to include a measuring unit 7, a deflection mirror 81, a reflection mirror 84, a concave mirror 85, and the like. The optometry apparatus 100 is provided with a subjective measuring unit. For example, the subjective measuring unit is configured to include a measuring unit 7, a deflection mirror 81, a reflection mirror 84, a concave mirror 85, and the like. The objective measuring unit and the subjective measuring unit are not limited to this configuration. For example, a configuration without the reflection mirror 84 may be used. In this case, the light flux from the measuring unit 7 may be irradiated from an oblique direction with respect to the optical axis L of the concave mirror 85 after passing through the deflection mirror 81. For example, a configuration having a half mirror may be used. In this case, the light flux from the measuring unit 7 may be irradiated from an oblique direction with respect to the optical axis L of the concave mirror 85 via the half mirror.

For example, the deflection mirror 81 includes a left eye deflection mirror 81L and a right eye deflection mirror 81R, which are provided as a pair on left and right, respectively. For example, the deflection mirror 81 is disposed between the calibration optical system 60 and the subject eye E. That is, the calibration optical system 60 in the present example includes a left eye calibration optical system and a right eye calibration optical system, which are provided as a pair on left and right, the left eye deflection mirror 81L is disposed between the left eye calibration optical system and the left eye EL, and the right eye deflection mirror 81R is disposed between the right eye calibration optical system and the right eye ER. For example, the deflection mirror 81 is preferably disposed at a pupil conjugate position.

For example, the left eye deflection mirror 81L reflects the light flux projected from the left eye measuring unit 7L and guides the light flux to the left eye EL. For example, the left eye deflection mirror 81L reflects the fundus reflection light flux from the left eye EL and guides the fundus reflection light flux to the left eye measuring unit 7L. For example, the right eye deflection mirror 81R reflects the light flux projected from the right eye measuring unit 7R and guides the light flux to the right eye ER. For example, the right eye deflection mirror 81R reflects the fundus reflection light flux from the right eye ER and guides the fundus reflection light flux to the right eye measuring unit 7R.

For example, the deflection mirror 81 is rotationally moved by the drive unit 82. For example, when the deflection mirror 81 is rotationally moved, it is possible to deflect an apparent light flux for forming an image of the target light flux in front of the subject eye and to optically correct the formation position of the image of the target light flux. For example, the drive unit 82 is configured to include a motor or the like. For example, the drive unit 82 rotates the deflection mirror 81 about a rotation axis in the horizontal direction (X direction) and a rotation axis in the vertical direction (Y direction). That is, the drive unit 82 rotates the deflection mirror 81 in the XY directions. The deflection mirror 81 may be rotated in either the horizontal direction or the vertical direction. For example, the drive unit 82 includes a drive unit 82L for driving the left eye deflection mirror 81L and a drive unit 82R for driving the right eye deflection mirror 81R.

In the present example, the configuration using the deflection mirror 81 as the deflection member that reflects and guides the light flux projected from the measuring unit 7 to the subject eye E is described as an example, but the configuration is not limited thereto. The deflection member may be able to reflect and guide the light flux projected from the measuring unit 7 onto the subject eye E, and may be a prism, a lens, or the like.

For example, a plurality of deflection mirrors 81 may be provided in each of the left eye optical path and the right eye optical path. For example, there is a configuration in which two deflection mirrors are provided in each of the left eye optical path and the right eye optical path (for example, a configuration in which two deflection mirrors are provided in the left eye optical path, and the like). In this case, one deflection mirror may be rotated in the X direction and the other deflection mirror may be rotated in the Y direction. For example, when the deflection mirror 81 is rotationally moved, it is possible to deflect an apparent light flux for forming an image of the target light flux in front of the subject eye and to optically correct the formation position of the image of the target light flux.

For example, the concave mirror 85 guides the target light flux passed through the calibration optical system 60 to the subject eye E, and forms an image of the target light flux passed through the calibration optical system 60 in front of the subject eye E. For example, the concave mirror 85 is shared by the left eye measuring unit 7L and the right eye measuring unit 7R. For example, the concave mirror 85 is shared by the left eye optical path including the left eye calibration optical system and the right eye optical path including the right eye calibration optical system. That is, the concave mirror 85 is disposed at a position where both the left eye optical path including the left eye calibration optical system and the right eye optical path including the right eye calibration optical system pass. As a matter of course, the concave mirror 85 may not be configured to be shared by the left eye optical path and the right eye optical path. That is, a concave mirror may be configured to be provided in each of the left eye optical path including the left eye calibration optical system and the right eye optical path including the right eye calibration optical system. For example, the concave mirror 85 guides the target light flux passed through the calibration optical system 60 to the subject eye E, and forms an image of the target light flux passed through the calibration optical system 60 in front of the subject eye E.

The deflection mirror 81 is driven by a drive unit 83 (for example, a motor or the like). For example, the drive unit 83 includes a left drive unit 83L for driving the left eye deflection mirror 81L and a right drive unit 83R for driving the right eye deflection mirror 81R. Each deflection mirror is moved in the X direction by driving the drive unit 83. For example, by moving the left eye deflection mirror 81L and the right eye deflection mirror 81R, the distance between the left eye deflection mirror 81L and the right eye deflection mirror 81R is changed, and the distance in the X direction between the left eye optical path and the right eye optical path can be changed according to the pupillary distance of the subject eye E.

The measuring unit 7 is driven by a drive unit 9 (for example, a motor or the like). For example, the drive unit 9 includes a left drive unit 9L for driving the left eye measuring unit 7L and a right drive unit 9R for driving the right eye measuring unit 7R. By driving the drive unit 9, each measuring unit moves in the X direction. For example, by moving the left eye measuring unit 7L and the right eye measuring unit 7R, the distance between each measuring unit and the deflection mirror 81 changes, and the presentation position in the Z direction of the target light flux from each measuring unit is changed. As a result, the target light flux corrected by the calibration optical system 60 is guided to the subject eye E, and the measuring unit 7 can be adjusted in the Z direction so that an image of the target light flux corrected by the calibration optical system 60 is formed on the fundus of the subject eye E.

### <Optical Path between Objective Measuring Unit and Subjective Measuring Unit>

The optical path of the objective measuring unit will be described by taking the left eye optical path as an example. The right eye optical path has the same configuration as the left eye optical path. The measurement light flux emitted from the light source 11 of the projection optical system 10a reaches the left eye EL via each optical member. For example, the measurement light flux is guided from the left eye measuring unit 7L to the left eye deflection mirror 81L by sequentially passing through the optical members from the relay lens 12 to the dichroic mirror 29. Furthermore, the target light flux is reflected by the left eye deflection mirror 81L and guided to the left eye EL via the reflection mirror 84 and the concave mirror 85. A spot-like point light source image is formed on the fundus of the left eye EL. At this time, the prism 15 that rotates around the optical axis eccentrically rotates the pupil projected image of the hole unit in the hole mirror 13 (projected light flux on the pupil) at high speed.

The measurement light flux is reflected and emitted from the fundus of the subject eye E, and is guided to the left eye measuring unit 7L via the concave mirror 85, the reflection mirror 84, and the deflection mirror 81. Furthermore, when the light is reflected by the dichroic mirror 29 and the dichroic mirror 35, condensed by the objective lens 93, and condensed again on the opening of the light receiving diaphragm 18 via the prism 15 rotating at high speed and the optical members from the hole mirror 13 to the mirror 17, a ring-shaped image is formed on the imaging element 22 by the collimator lens 19 and the ring lens 20. By analyzing the ring-shaped image captured by the imaging element 22, the optical characteristics of the subject eye E can be objectively measured.

The optical path of the subjective measuring unit will be described by taking the left eye optical path as an example. The right eye optical path has the same configuration as the left eye optical path. The target light flux emitted from the display 31 of the subjective measurement optical system 25 reaches the left eye EL via each optical member. For example, the target light flux is guided from the left eye measuring unit 7L to the left eye deflection mirror 81L by sequentially passing through the optical members from the projection lens 33 to the dichroic mirror 29. Furthermore, the target light flux is reflected by the left eye deflection mirror 81L and guided to the left eye EL via the reflection mirror 84 and the concave mirror 85.

As a result, an image of the target light flux corrected by the calibration optical system 60 is formed on the fundus of the left eye EL with reference to the spectacle wearing position of the left eye EL (for example, approximately 12 mm from the corneal apex position). Therefore, the fact that the spherical power is adjusted by the calibration optical system (in the present example, driving of the drive mechanism 39) in front of the eye is equivalent to the fact that the astigmatism calibration optical system 63 is disposed in front of the eye. The subject can collimate in a natural state the image of the target light flux optically formed in front of the eye at a predetermined test distance via the concave mirror 85.

### <Control Section>

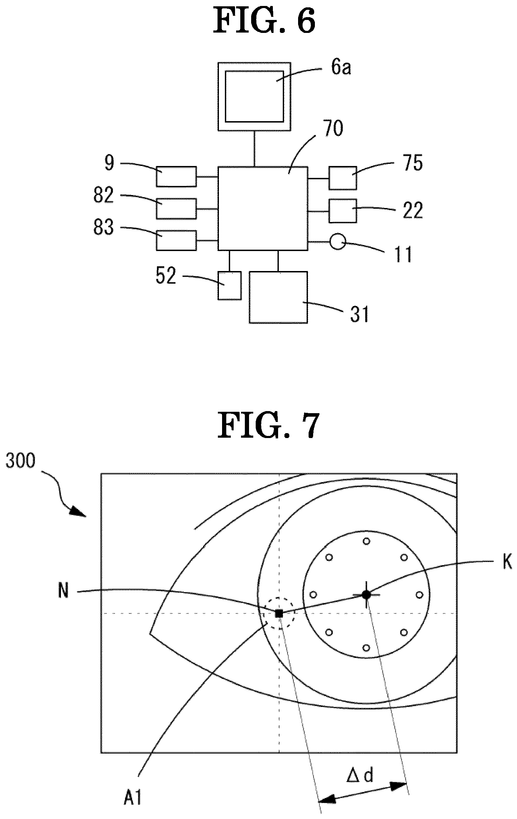

FIG. 6 is a diagram illustrating a control system of the optometry apparatus 100. The control section 70 is provided with a processor (CPU), a RAM, a ROM, and the like. For example, the CPU controls each member in the optometry apparatus 100. For example, the RAM temporarily stores various information. For example, the ROM stores various programs, the target, the initial value, and the like for controlling the operation of the optometry apparatus 100. The control section 70 may be configured to include a plurality of control sections (that is, a plurality of processors).

For example, various members such as the monitor 6a, the light source 11, the imaging element 22, the display 31, the imaging element 52, and the nonvolatile memory 75 (hereinafter referred to as memory 75) are electrically connected to the control section 70. For example, the control section 70 is electrically connected to the drive unit 9, the drive unit 82, the drive unit 83, the drive mechanism 39, and the like. For example, the memory 75 is a non-transitory storage medium that can retain stored content even when the power supply is interrupted. For example, a hard disk drive, a flash ROM, a USB memory, or the like can be used as the memory 75.

### <Control Operation>

A control operation of the optometry apparatus 100 will be described.

The examiner instructs the subject to cause his or her face to abut on the forehead rest 4 and the chin rest 5 and observe the presentation window 3. The examiner operates the switch unit 6b to operate the switch for starting the measurement on the subject eye E. Based on the operation signal from the switch unit 6b, the control section 70 performs the initial alignment with respect to the subject eye E, and then automatically proceeds the objective measurement and the subjective measurement.

### <Initial Alignment of Subject Eye>

The control section 70 causes the display 31 of the left eye measuring unit 7L to display a fixation target for fixating the left eye in response to an operation signal for starting measurement on the subject eye E. The display 31 of the right eye measuring unit 7R displays a fixation target for fixating the right eye. As a result, the left eye and the right eye are in a state of the binocular fusion of the fixation target.

The control section 70 projects an alignment index image by the first index projection optical system 45 and the second index projection optical system 46 of the left eye measuring unit 7L onto the cornea of the left eye. The control section 70 detects the deviation of the left eye measuring unit 7L from the left eye in the X direction, Y direction, and Z direction based on the alignment index image, and moves the left eye measuring unit 7L. Similarly, the control section 70 projects an alignment index image onto the cornea of the right eye, and moves the right eye measuring unit 7R based on the alignment index image.

FIG. 7 is an example of an anterior chamber image 300 of the left eye. For example, the alignment reference position N is set at the center position of the anterior chamber image 300 and coincident with the optical axis L4L. For example, a predetermined XY range with reference to the alignment reference position N is set as an alignment allowable range A1 for determining whether the alignment in the XY directions is appropriate. For example, a predetermined Z range with reference to the alignment reference position N is set as an alignment allowable range (not illustrated) for determining whether the alignment in the Z direction is appropriate.

The control section 70 detects the XY central coordinates in the alignment index image as the corneal apex position K, and detects whether the corneal apex position K is displaced from the alignment reference position N by obtaining the amount of deviation Δd between the corneal apex position K and the alignment reference position N. The control section 70 moves the left eye measuring unit 7L in the X direction and the Y direction so that the amount of deviation Δd is within the alignment allowable range A1. The control section 70 moves the left eye measuring unit 7L in the Z direction, so that the image ratio between the image interval of the infinite alignment index images and the image interval of the finite alignment index images is within an alignment allowable range (not illustrated). When the amount of deviation Δd and the image ratio fall within the allowable range, the movement of the left eye measuring unit 7L is stopped.

For example, the alignment between the left eye and the left eye measuring unit 7L and the alignment between the right eye and the right eye measuring unit 7R are also performed by the same control based on the alignment allowable range.

### <Objective Measurement>

When the initial alignment between the left eye and the left eye measuring unit 7L and the initial alignment between the right eye and the right eye measuring unit 7R are completed, the control section 70 automatically starts the objective measurements for the left eye and the right eye. For example, a fixation target is continuously presented to the left eye and the right eye, and the objective measurements of the left eye and the right eye are performed in parallel in a state where the left eye and the right eye are subjected to binocular fusion of the fixation target.

At this time, the control section 70 sets an alignment allowable range B1 for the objective measurement with reference to the alignment reference position N, and causes the left eye measuring unit 7L and the right eye measuring unit 7R to track (that is, tracking control is performed), based on the alignment allowable range B1. The alignment allowable range B1 for the objective measurement may be the same allowable range as the alignment allowable range A1 described above. In this case, the tracking control based on the alignment allowable range A1 is performed continuously from the initial alignment.

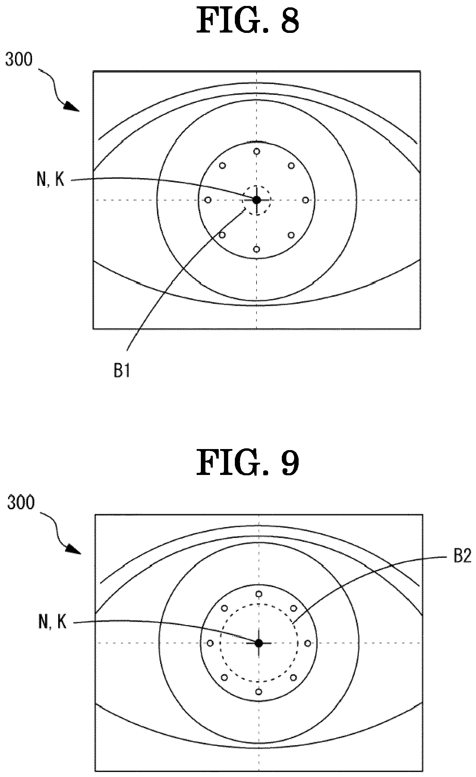

FIG. 8 is a diagram for describing the alignment allowable range B1 for the objective measurement. In the objective measurement, it is necessary to finely align the subject eye and each measuring unit. For example, when the face of the subject or the line of sight of the subject eye moves and the predetermined positional relationship between the subject eye and each measuring unit is disrupted, the size and shape of the ring image are changed consequently, and an appropriate measurement result is not obtained. In particular, in a case where each measuring unit is significantly deviated with respect to the subject eye, the ring image is significantly affected, and the measurement result is calculated as a value different from the actual eye refractive power. Therefore, the alignment allowable range B1 for the objective measurement is set as a narrower allowable range than the alignment allowable range B2 for the subjective measurement (described later). As an example, the range may be set within a range of ϕ 0.5 mm from the alignment reference position N. As a matter of course, the range may be different from ϕ 0.5 mm.

In the objective measurement, the preliminary measurement may be performed first, and the main measurement may be performed later. In the preliminary measurement of the left eye, the eye refractive power is measured with the fixation target disposed at a predetermined presentation distance. For example, the control section 70 disposes the display 31 of the left eye measuring unit 7L at an initial position that is optically sufficiently far away from the left eye and corresponds to the far point of the 0 D eye. The left eye is irradiated with the measurement light flux from the light source 11 of the projection optical system 10a, and the ring image captured by the imaging element 22 of the light receiving optical system 10b is subjected to image analysis. For example, the control section 70 thins the ring image to obtain the eye refractive power in each meridian direction, performs predetermined processing on the eye refractive power, and acquires at least the spherical power (spherical power in the preliminary measurement).

Subsequently, fog is added to the left eye. For example, the control section 70 disposes the display 31 of the left eye measuring unit 7L at the fog start position where the left eye is focused, according to the preliminary measurement of the spherical power of the left eye. The left eye is able to observe the fixation target clearly. Thereafter, the control section 70 moves the fixation target from the fog start position. The left eye is no longer focused on the fixation target and fog is added. As a result, the adjustment of the left eye is released and the eye refractive power approaches the true value.

In the main measurement on the subject eye E, the eye refractive power is measured with fog added to the left eye. For example, the control section 70 causes the imaging element 22 to continuously capture ring images at predetermined timings. For example, the control section 70 performs addition processing on the ring image to reduce noise light, acquires the eye refractive power of the left eye (spherical power, cylindrical power, and astigmatism axis angle in the main measurement) by the same method as the preliminary measurement, and stores the eye refractive power in the memory 75.

For example, the objective eye refractive power measurement of the left eye and the objective eye refractive power measurement of the right eye are performed by similar control.

During the preliminary measurement and the main measurement on the left eye and the right eye, tracking control based on the alignment allowable range B1 for the objective measurement is performed for each of the left eye and the right eye. The control section 70 detects from the anterior chamber image 300 at any time whether the amount of deviation Δd between the corneal apex position K of the left eye and the alignment reference position N is within the alignment allowable range B1. In a case where the amount of deviation Δd is deviated from the alignment allowable range B1, the control section 70 restarts the alignment and moves the left eye measuring unit 7L. When the amount of deviation Δd enters the alignment allowable range B1, the movement of the left eye measuring unit 7L is stopped. As for the right eye, the right eye measuring unit 7R is moved and stopped based on the amount of deviation Δd as needed.

Since the presentation positions of the fixation target for the left eye and the right eye are adjusted according to the tracking control of the control section 70, the fixation target may appear to move to the subject. However, since the time from the start of the objective measurement to obtaining the measurement results is short, it is unlikely to feel the movement of the fixation target. Therefore, in the objective measurement, even when the alignment allowable range B1 is set narrowly, it is unlikely to feel annoyance and fatigue due to movement of the fixation target, and an objective eye refractive power (objective value) can be appropriately obtained.

### <Subjective Measurement>

When acquiring the objective eye refractive powers of the left eye and the right eye, the control section 70 automatically starts the subjective measurement of the left eye and the right eye. For example, the subjective measurement of the left eye and the subjective measurement of the right eye are performed in order.

The control section 70 turns off the display 31 of each measuring unit and stops presenting the fixation target. The control section 70 sets the spherical power, the cylindrical power, and the astigmatism axis angle based on the objective eye refractive power of the left eye. For example, the spherical power of the left eye may be corrected by moving the display 31 in the direction of the optical axis L2. For example, at least one of the cylindrical power and the astigmatism axis angle of the left eye may be corrected by rotating the cylindrical lenses 61a and 61b around the axis of the optical axis L2. As a result, the left eye is corrected with a predetermined diopter value (for example, 0 D). Similarly, the control section 70 corrects the right eye with a predetermined diopter value (for example, 0 D) based on the objective eye refractive power of the right eye.

Subsequently, the control section 70 causes the display 31 of the left eye measuring unit 7L to display a predetermined test target and a background image, and causes the display 31 of the right eye measuring unit 7R to display the background image. For example, the background image may be an image with a white background. As a matter of course, an image other than a white background may be used. As a result, the test target can be presented only to the left eye while creating a state of binocular vision without obstructing the front of the left eye and the right eye.

Furthermore, after presenting the test target or background image to the subject eye E, the control section 70 detects whether the amount of deviation Δd between the corneal apex positions K of the left eye and the right eye and the alignment reference position N is within the alignment allowable range B1 for the objective measurement. In a case where the amount of deviation Δd is deviated from the alignment allowable range B1, the alignment is restarted and the left eye measuring unit 7L and the right eye measuring unit 7R are moved. When the amount of deviation Δd enters the alignment allowable range B1, the movement of the left eye measuring unit 7L and the right eye measuring unit 7R is stopped.

In the subjective measurement on the subject eye E, since the test target is switched to the subject eye E or the presentation distance of the test target is changed, the time from the start of the subjective measurement to obtaining the measurement result is longer than in the objective measurement, and the frequency of movement of the face of the subject and the line of sight of the subject eye E increases. In a case where the subjective measurement is proceeded while the alignment allowable range B1 for the objective measurement is set, the subject is likely to feel the movement of the test target, and there is a possibility that an appropriate measurement result may not be obtained due to annoyance or fatigue. Therefore, once the subject eye and each measuring unit are finely aligned by alignment based on the alignment allowable range B1 for the objective measurement, the control section 70 changes the setting to the alignment allowable range B2 for the subjective measurement. The subjective eye refractive powers of the left eye and the right eye are acquired while the left eye measuring unit 7L and the right eye measuring unit 7R are caused to track based on the alignment allowable range B2 for the subjective measurement.

FIG. 9 is a diagram for describing the alignment allowable range B2 for subjective measurement. In the subjective measurement on the subject eye E, the measurement can be proceeded as long as each of the measuring units is adjusted to the extent that the subject eye E can recognize the test target. More specifically, even when the face of the subject or the line of sight of the subject eye E moves and the predetermined positional relationship between the subject eye E and the measuring unit 7 is disrupted, if a part of the test target does not appear to be missing, deviation of each measuring unit is allowed. Therefore, the alignment allowable range B2 for the subjective measurement is set to have a wider allowable range than the alignment allowable range B1 for the objective measurement. As an example, with the alignment reference position N as a reference, any allowable range within ϕ 2.0 mm to ϕ 4.0 mm may be set. As a matter of course, the allowable range may be smaller than ϕ 2.0 mm or may be larger than ϕ 4.0 mm.

The examiner operates the switch unit 6b to check whether the correction power for correcting the left eye is appropriate while switching the visual acuity value of the test target to be presented to the left eye. For example, the subject is asked about the direction of the test target, and when the subject answers correctly, the visual acuity value is switched to one step higher value (that is, lower value), and when the subject answers incorrectly, the visual acuity value is switched to one step lower value (that is, higher value). The control section 70 changes the test target displayed on the display 31 based on the change signal from the switch unit 6b. In a case where the correction power for correcting the left eye EL is inappropriate, the correction power is changed. As a result, the subjective eye refractive power (subjective value) of the left eye is acquired.

For example, similar to the measurement of subjective eye refractive power of the left eye, in the state of binocular vision of the left eye and the right eye, by presenting the test target only to the right eye, and checking whether the correction power is appropriate while switching the visual acuity value of the test target presented to the right eye, the subjective eye refractive power (subjective value) of the right eye is acquired.

During the subjective measurement on the left eye or the right eye, tracking control based on the alignment allowable range B2 for the subjective measurement is performed for each of the left eye and the right eye. The control section 70 detects from the anterior chamber image 300 at any time whether the amount of deviation Δd between the corneal apex position K of the left eye and the alignment reference position N is within the alignment allowable range B2. In a case where the line of sight of the subject eye E moves and the amount of deviation Δd is deviated from the alignment allowable range B2, the control section 70 restarts the alignment and moves the left eye measuring unit 7L and the right eye measuring unit 7R. When the amount of deviation Δd enters the alignment allowable range B2, the movement of the left eye measuring unit 7L is stopped. As for the right eye, the right eye measuring unit 7R is moved and stopped based on the amount of deviation Δd as needed.

When the presentation positions of the test target for the left eye and the right eye are adjusted according to the tracking control of the control section 70, the test target may appear to move to the subject. However, by setting the alignment allowable range B2 for the subjective measurement to be wide, the left eye and the right eye are unlikely to deviate from the alignment allowable range B2, and the number of times tracking control is performed is reduced. Therefore, even in the subjective measurement, it is unlikely to feel annoyance and fatigue due to movement of the test target, and an appropriate measurement result can be obtained.

### <Measurement of Binocular Vision Function by Binocular Fusion State>

In the subjective measurement on the subject eye E, the binocular vision function can be measured by being subjected to the binocular fusion for the left eye and the right eye while creating a state of binocular vision without blocking the front of the left eye and the right eye. For example, the binocular vision function of the subject eye E may be at least one of information such as binocular balance, stereoscopic vision, oblique position, and anisotropic vision. Based on the operation signal from the switch unit 6b, the control section 70 displays the background image and the predetermined test target on each of the display 31 of the left eye measuring unit 7L and the display 31 of the right eye measuring unit 7R. That is, the same background image and the same test target are presented to the left eye and the right eye.

In this manner, even in a case where the left eye and the right eye are subjected to the binocular fusion in the subjective measurement on the subject eye E, tracking control based on the alignment allowable range B2 for the subjective measurement is performed for the left eye and the right eye. For example, when the presentation positions of the test target for the left eye and the right eye are finely adjusted, it is likely to feel annoyance and fatigue due to the test target appearing to move, and there is a possibility that the binocular fusion is canceled and the test target may appear to be separated. By setting the alignment allowable range B2 for the subjective measurement to a wide allowable range, it is possible to suppress the cancellation of the fusion state due to binocular vision and to make it unlikely to separate the test targets.

### <Objective Measurement during Subjective Measurement>

In the subjective measurement on the subject eye E, the objective measurement on the subject eye E can be performed in parallel. In other words, while subjectively measuring the eye refractive power of the subject eye E, the eye refractive power of the subject eye E can be objectively measured. For example, adjustment information of the subject eye E can be acquired from the change in the eye refractive power (first eye refractive power) acquired by the objective measurement on the subject eye E and the eye refractive power (second eye refractive power) acquired by the objective measurement during the subjective measurement on the subject eye E. The adjustment information of the subject eye E may be information related to the adjustment function of the subject eye E. As an example, it may be information such as whether it is in an adjusted state, and how the adjusted state is changed.

In this manner, when the objective measurement and the subjective measurement on the subject eye E are performed in parallel, the alignment allowable range B2 for the subjective measurement is set. In other words, when performing both the objective measurement and the subjective measurement, priority is given to the alignment allowable range B2 for the subjective measurement. For example, when the alignment allowable range B1 for the objective measurement is set for the objective measurement optical system, the second eye refractive power cannot be acquired when the amount of deviation Δd exceeds the alignment allowable range B1 but continues to be within the alignment allowable range B2. By setting the alignment allowable range for the objective measurement and the subjective measurement to the same allowable range, the second eye refractive power can be acquired smoothly.

For example, the control section 70 detects whether the amount of deviation Δd is within the alignment allowable range B2, and moves the left eye measuring unit 7L and the right eye measuring unit 7R so that the amount of deviation Δd falls within the alignment allowable range B2. The control section 70 acquires the second eye refractive power at least at any timing (for example, the timing of the start of the subjective measurement, the timing of switching the test target, the timing of switching the correction power, and the like) from the start of the subjective measurement to the end of the subjective measurement. For example, the adjustment information may be acquired by obtaining the difference between the first eye refractive power and the second eye refractive power.

Hereinbefore, as described above, for example, the optometry apparatus of the present example performs the first alignment processing based on the first alignment allowable range for determining the deviation between the subject eye and the objective measurement optical system, and performs the second alignment processing based on the second alignment allowable range for determining the deviation between the subject eye and the subjective measurement optical system, in which the second alignment allowable range has a wider allowable range than the first alignment allowable range. For example, in the objective measurement, since the deviation between the subject eye and the objective measurement optical system is finely adjusted based on the first alignment allowable range, the measurement results can be obtained with high accuracy. For example, in the subjective measurement, by allowing the deviation between the subject eye and the subjective measurement optical system to some extent based on the second alignment allowable range, the frequency of the target appearing to move is reduced, so that good measurement results can be obtained.

For example, the optometry apparatus of the present example adjusts the relative positional relationship between the subject eye and the objective measurement optical system as the first alignment processing, and adjusts the relative positional relationship between the subject eye and the subjective measurement optical system as the second alignment processing. As a result, each optical system can be quickly disposed at an appropriate position according to the deviation between the subject eye, the objective measurement optical system, and the subjective measurement optical system.

For example, the optometry apparatus of the present example changes between the first alignment allowable range corresponding to objective measurement and the second alignment allowable range corresponding to subjective measurement, when measurement on the subject eye is switched from one of the objective measurement by the objective measurement optical system and the subjective measurement by the subjective measurement optical system to the other. As a result, each measurement can be proceeded smoothly in a series of objective measurement and subjective measurement on the subject eye. For example, in the objective measurement, the measurement proceeds smoothly by moving the objective optical system with high accuracy. For example, in the subjective measurement, the subject is unlikely to feel annoyance or fatigue due to the test target appearing to move, and the measurement proceeds smoothly.

For example, the optometry apparatus of the present example sets the second alignment allowable range for the subjective measurement in a case where the optical characteristics of the subject eye is objectively measured while the optical characteristics of the subject eye is subjectively measured. For example, in such a configuration, when the first alignment allowable range for the objective measurement is set (in other words, priority is given to the first alignment allowable range), problems such as annoyance due to the movement of the test target cannot be solved. For example, in such a configuration, when the alignment allowable ranges for the objective measurement and subjective measurement are different, there is a possibility that the measurement result of the objective measurement cannot be acquired when the range is deviated from the first alignment allowable range of the objective measurement but is within the second alignment allowable range of the subjective measurement. By setting the second alignment allowable range, the measurement results of the objective measurement and the subjective measurement can be acquired satisfactorily.

For example, the optometry apparatus of the present example includes a measurement unit including the objective measurement optical system and the subjective measurement optical system, and having a pair of the left eye measurement unit and the right eye measurement unit, in which the left eye measurement unit is aligned with the left eye and the right eye measurement unit is aligned with the right eye, respectively. As a result, it is possible to perform not only monocular measurement using one of the left eye measurement unit and the right eye measurement unit, but also binocular measurement using both the left eye measurement unit and the right eye measurement unit.

For example, in the optometry apparatus of the present example, the left eye measurement unit projects the target light flux onto the left eye, and the right eye measurement unit projects the target light flux onto the right eye, the left eye and the right eye being subjected to a binocular fusion. For example, in a case of measuring the binocular vision function of the subject eye, when the alignment allowable range for the subjective measurement is narrow and the test target appears to move, there is a possibility that the fusion state of both eyes is canceled and the left and right test targets appears to be separated, which is likely to feel annoyance. However, by setting a wide alignment allowable range for the subjective measurement, the separation of test targets can be suppressed, and measurement results can be acquired with high accuracy.

### <Transformation Example>

In the present example, the configuration in which the face of the subject is fixed by the forehead rest 4 and the chin rest 5 is described as an example, but the configuration is not limited thereto. For example, the face of the subject may be fixed only by the forehead rest 4. For example, in a subjective measurement that proceeds based on the answer of the subject, the subject eye E is likely to be moved particularly in the Y direction when the subject speaks with the jaw abutted on the chin rest 5. Such movement in the Y direction may be suppressed by using only the forehead rest 4 without using the chin rest 5.

In the present example, the configuration in which the alignment allowable range B1 in the XY direction for the objective measurement and the alignment allowable range B2 in the XY direction for the subjective measurement on the subject eye E are set is described as an example, but the configuration is not limited thereto. For example, since the face of the subject abuts on the forehead rest 4 and the chin rest 5 to be unlikely to move, in the objective measurement and the subjective measurement, the alignment allowable range may be set only in the XY directions so as to correspond to at least the movement of the line of sight. However, since the face of the subject may move, an alignment allowable range in the Z direction may be set. In this case, the alignment allowable range in the Z direction for the subjective measurement may be set wider than the alignment allowable range in the Z direction for the objective measurement.

In the present example, the configuration in which the alignment allowable range B1 for the objective measurement and the alignment allowable range B2 for the subjective measurement are set to fixed values in advance is described as an example, but the configuration is not limited thereto. For example, the alignment allowable range B1 for the objective measurement may be changed to any value. For example, the alignment allowable range B2 for the subjective measurement may be changed to any value. In the present example, at least the alignment allowable range B2 may be changed to any value.

For example, when the alignment allowable range is larger than the pupil diameter of the subject eye E, even though the measuring unit 7 is moved so that the corneal apex position K of the subject eye E is within the alignment allowable range, a positional relationship is such that the target light flux from the subjective measurement optical system is projected outside the pupil diameter, and there is a possibility that the subject eye E cannot recognize the test target. In particular, in a case where the subject eye E is an eye with a small pupil, the alignment allowable range B1 for the objective measurement is set to a narrow allowable range, so that it is unlikely to be a problem, but the alignment allowable range B2 for the subjective measurement is set to a wide allowable range, so that it is likely to be a problem. Therefore, the examiner changes the alignment allowable range B2 to any value according to the pupil diameter of the subject eye E, and the like, it is possible to acquire measurement results with high accuracy while suppressing the annoyance of the test target appearing to move in the subjective measurement.

The alignment allowable range B2 for the subjective measurement may be automatically changed to an appropriate value according to the pupil diameter of the subject eye E. In this case, the control section 70 acquires the pupil diameter of the subject eye E, and changes the alignment allowable range B2 based on the pupil diameter. For example, the pupil diameter of the subject eye E may be acquired by detecting the pixel position corresponding to the pupil or the pixel position corresponding to the iris based on the luminance information of the anterior chamber image 300. For example, the pupil diameter of the subject eye E may be acquired when the examiner inputs by operating the switch unit 6b. The control section 70 may change the alignment allowable range B2 by setting a predetermined alignment allowable range associated with the pupil diameter.

In the present example, in a case where the amount of deviation Δd between the subject eye E and the alignment reference position N is deviated from the alignment allowable range B2 during the subjective measurement on the subject eye E, the configuration in which the measuring unit 7 is moved such that the amount of deviation Δd is within the alignment allowable range B2 is described as an example, but the configuration is not limited thereto. For example, in a case where the amount of deviation Δd is deviated from the alignment allowable range B2, the measuring unit 7 may be moved so that the amount of deviation Δd is within the alignment allowable range B1. That is, the alignment allowable range B2 for the subjective measurement may be used to detect whether the amount of deviation Δd is within the alignment allowable range, and the alignment allowable range B1 for the objective measurement may be used when the tracking control is performed (restarted the alignment) based on this detection result. As a result, since the time from the alignment of the subject eye E and the measuring unit 7 to the occurrence of deviation again increases, the frequency of tracking control can be reduced as a result.

In the present example, a configuration in which the eye refractive power is measured for each eye while the left eye and the right eye of the subject eye E are binocularly viewed is described as an example, but the configuration is not limited thereto. In the present example, the eye refractive power of the right eye can be measured while the left eye is shielded by a shield or the like, and then the eye refractive power of the left eye can be measured while the right eye is shielded by the shield or the like. In other words, it is possible to perform measurement on the other eye in the monocular state of either the left eye or the right eye. Even in such a case, by setting the alignment allowable range B2 for the subjective measurement to a wider allowable range than the alignment allowable range B1 for the objective measurement on the subject eye E, movement of the test target in the subjective measurement is unlikely to be felt, and the measurement results can be acquired with high accuracy.

In the present example, during the period from the end of the objective measurement on the subject eye E and the stop of the presentation of the fixation target to the start of the subjective measurement and the presentation of the test target, tracking control based on the alignment allowable range B1 for the objective measurement may be continuously performed. In this case, control for switching between execution and stop of tracking control, such as stopping tracking control when the fixation target is turned off and restarting tracking control when the test target is turned on, is suppressed, and complication of control is suppressed.

In the present example, during the period from the end of the objective measurement on the subject eye E and the stop of the presentation of the fixation target to the start of the subjective measurement and the presentation of the test target, tracking control based on the alignment allowable range B1 for the objective measurement may be stopped. In this case, since the movement of the measuring unit 7 is stopped at a stage when the fixation target is turned off, the measuring unit 7 does not follow the subject eye even when the line of sight of the subject eye moves significantly before the test target is presented. When the subject eye visually recognizes the test target, since the line of sight returns to the original position (position at a stage when the fixation target is turned off), the amount of movement of the measuring unit 7 is reduced when the tracking control based on the alignment allowable range B1 is restarted, and as a result, the subjective measurement can be easily performed.

In the present example, by moving the measuring unit 7 and the concave mirror 85 with respect to the subject eye while maintaining the positional relationship between the measuring unit 7 and the concave mirror 85, and by moving the measuring unit 7 with respect to the concave mirror 85, the projection optical system 10a and the light projecting optical system 30 may be configured to be aligned with the subject eye (for details, refer to JP2022-179009A). In this case, as in the present example, the alignment allowable range B2 for the subjective measurement may be set to have a wider allowable range than the alignment allowable range B1 for the objective measurement.

## Claims

1. An optometry apparatus that measures optical characteristics of a subject eye, comprising:
an objective measurement optical system including a first light projecting optical system that projects a measurement light flux onto a fundus of the subject eye, and a light receiving optical system in which a detector detects a reflection light flux, reflected by the fundus, of the measurement light flux, and for objectively measuring the optical characteristics of the subject eye;
a subjective measurement optical system including a second light projecting optical system that projects a target light flux toward the subject eye, and a calibration optical system that is disposed in an optical path of the second light projecting optical system and changes optical characteristics of the target light flux, and for subjectively measuring the optical characteristics of the subject eye;
a first control means configured to perform first alignment processing based on a first alignment allowable range for determining a deviation between the subject eye and the objective measurement optical system; and
a second control means configured to perform second alignment processing based on a second alignment allowable range for determining a deviation between the subject eye and the subjective measurement optical system,
wherein the second alignment allowable range has a wider allowable range than the first alignment allowable range.

2. The optometry apparatus according to claim 1,
wherein the first control means is configured to adjust a relative positional relationship between the subject eye and the objective measurement optical system as the first alignment processing, and
the second control means is configured to adjust a relative positional relationship between the subject eye and the subjective measurement optical system as the second alignment processing.

3. The optometry apparatus according to claim 1 or 2, further comprising:
a change control means configured to change between the first alignment allowable range corresponding to objective measurement and the second alignment allowable range corresponding to subjective measurement, when measurement on the subject eye is switched from one of the objective measurement by the objective measurement optical system and the subjective measurement by the subjective measurement optical system to the other.

4. The optometry apparatus according to claim 3,
wherein the change control means sets the second alignment allowable range, in a case where the optical characteristics of the subject eye is objectively measured by the objective measurement optical system while the optical characteristics of the subject eye is subjectively measured by the subjective measurement optical system.

5. The optometry apparatus according to any one of claims 1 to 4, further comprising:
a measurement unit including the objective measurement optical system and the subjective measurement optical system, and having a pair of a left eye measurement unit and a right eye measurement unit,
wherein the left eye measurement unit is aligned with a left eye and the right eye measurement unit is aligned with a right eye, respectively.

6. The optometry apparatus according to claim 5,
wherein the left eye measurement unit projects the target light flux onto the left eye and the right eye measurement unit projects the target light flux onto the right eye, the left eye and the right eye being subjected to a binocular fusion.

7. The optometry apparatus according to any one of claims 1 to 6, further comprising:
a shared optical member shared by an optical path of the objective measurement optical system and an optical path of the subjective measurement optical system, and for optically presenting an image of the target light flux to the subject eye at a predetermined test distance by guiding the target light flux corrected by the calibration optical system to the subject eye.

8. An optometry program executed by a processor of an optometry apparatus that includes: an objective measurement optical system including a first light projecting optical system that projects a measurement light flux onto a fundus of a subject eye, and a light receiving optical system, in which a detector detects a reflection light flux, reflected by the fundus, of the measurement light flux, and for objectively measuring optical characteristics of the subject eye; and a subjective measurement optical system including a second light projecting optical system that projects a target light flux toward the subject eye, and a calibration optical system that is disposed in an optical path of the second light projecting optical system and changes optical characteristics of the target light flux, and for subjectively measuring the optical characteristics of the subject eye, and measures the optical characteristics of the subject eye, the optometry program comprising instructions which, when executed by the processor of the optometry apparatus, cause the optometry apparatus to perform:
a first control step of performing first alignment processing based on a first alignment allowable range for determining a deviation between the subject eye and the objective measurement optical system; and
a second control step of performing second alignment processing based on a second alignment allowable range for determining a deviation between the subject eye and the subjective measurement optical system,
wherein the second alignment allowable range has a wider allowable range than the first alignment allowable range.
